Europäisches Patentamt

European Patent Office

Office européen des brevets

(11) Veröffentlichungsnummer: **0 216 952**
**A1**

## (12) EUROPÄISCHE PATENTANMELDUNG

(21) Anmeldenummer: 85112573.2

(22) Anmeldetag: 04.10.85

(51) Int. Cl.⁴: **A61F 9/00**

(43) Veröffentlichungstag der Anmeldung:
**08.04.87 Patentblatt 87/15**

(84) Benannte Vertragsstaaten:
**DE FR IT NL**

(71) Anmelder: **Erbe Elektromedizin GmbH.**
**Ebertstrasse 35**
**D-7400 Tübingen(DE)**

(72) Erfinder: **van Gerven, Johannes Theodorus**
**Maria**
**Ebertstrasse 29**
**D-7400 Tübingen(DE)**

(74) Vertreter: **Endlich, Fritz, Dipl.-Phys.**
**Postfach 1326 Blumenstrasse 8**
**D-8034 Germering(DE)**

(54) **Einrichtung zur subretinalen Drainage.**

㊲ Einrichtung zur Drainage subretinaler Flüssigkeitsansammlungen (2) für die Anwendung in der Augenheilkunde bestehend aus einem Saugnapf, (5) welcher von außen an den Augapfel (4) angesaugt wird und einer elektrisch isolierten Nadelelektrode (7), welche durch ein automatisches Antriebsaggregat angetrieben wird, wobei sowohl die relative Lage der Nadelspitze der Nadelelektrode als auch deren Hub einstellbar sind. Der Saugnapf verhindert eine Eindellung der Sclera (3) während des Einstechens der Nadelspitze (8) die Sclera. Die Nadelspitze ist elektrisch nicht isoliert und kann mit Gleichspannung versorgt werden, so daß eine elektrolytische Verätzung der durch Einstich verursachten Perforation der Sclera ausgeführt werden kann.

Fig.1

## Einrichtung zur subretinalen Drainage

Die Erfindung betrifft eine Einrichtung zur subretinalen Drainage für die Anwendung in der Augenheilkunde.

Eine medizinische Indikation zur subretinalen Drainage sind lokale Flüssigkeitsansammlungen zwischen der Retina und Choroidea und der Sclera infolge einer Ablatio retinae.

Es ist bekannt, diese Drainage beispielsweise mittels einer Injektionskanüle durch die Sclera hindurch anzulegen. Diese Technik ist insofern problematisch, als die Einstichtiefe der Kanüle nur unbefriedigend kontrollierbar ist und daß sich die durch den Einstich entstehende Perforation der Sclera nach Entfernen der Kanüle elastisch schließt und anschließend relativ schnell verheilt bzw. vernarbt, und zwar schneller als für den Heilungsvorgang erforderlich.

Um zu erreichen, daß die Retina, inklusive Choroidea, sich nach der Drainage möglichst ganzflächig und ausreichend fest an die Sclera anlegt, muß die Drainage vollständig erfolgen, was durch den Strömungswiderstand der sich nach dem Entfernen der Kanüle wieder verschließenden Perforation verhindert wird.

Es wurde versucht, die Perforation der Sclera langfristiger anzulegen, indem mittels einer Nadelelektrode und hochfrequentem elektrischem Strom das Sclera-Gewebe im Bereich des Einstiches der Nadelelektrode thermisch koaguliert wurde. Diese Methode führte jedoch nicht zu dem erhofften Erfolg. Hochfrequenter elektrischer Strom wird mit gegenteiliger Wirkung in der Augenheilkunde dafür verwendet, feine, gezielte Narben in der Grenzschicht zwischen Retina und Sclera zu setzen, wodurch Netzhautablösungen wieder fixiert werden. Heute wird mehr und mehr Laser für diesen Zweck verwendet. Da Narbenbildung die Perforationen verschließen, ist der hochfrequente Strom hierfür nicht geeignet.

Es ist jedoch bekannt, daß Gewebeverätzungen mittels elektrischen Gleichstromes, vorzugsweise an der Anode, relativ langsam vernarben.

Ein weiteres Problem beim Einstechen einer Kanüle in die Sclera besteht darin, daß die Sclera an der Stelle, wo die Kanüle gegen die Sclera gedrückt wird, eine Eindellung entsteht bevor die Kanüle in die Sclera einsticht. Sobald aber der Einstich erfolgt, bildet sich die Eindellung plötzlich zurück, so daß die Einstichtiefe der Kanüle nicht ausreichend kontrollierbar ist. Sticht die Kanüle zu tief durch die Sclera, dann besteht die Gefahr, daß die Choroidea und die Retina ebenfalls durchstochen oder zumindest verletzt werden.

**Es ist Aufgabe der Erfindung,** eine Einrichtung zur subretinalen Drainage von Flüssigkeitsansammlungen zwischen Retina und Sclera zu schaffen, mittels welcher der Ophthalmologe problemlos eine effiziente und ausreichend langfristige Drainage anlegen kann.

Der Erfindung liegt die Beobachtung zugrunde, daß bei jedem Versuch, eine Nadel in ein biologisches Gewebe einzustechen, und das gilt auch für die Sclera, dieses Gewebe infolge seiner mehr oder weniger großen Elastizität von der Nadelspitze eingedrückt wird, so daß eine Delle entsteht. Sobald der Druck der Nadelspitze gegen das Gewebe ausreichend hoch ist, sticht die Nadelspitze plötzlich weitgehend unkontrolliert tief in das Gewebe ein. Um diese Eindellung während des Einstechens der Nadel zu vermeiden, wird ein kleiner Saugnapf verwendet, welcher an die Kugeloberfläche des Augapfels angeformt ist und in dessen Zentrum ein kleines Loch angeordnet ist, durch welches die Nadel zur Perforation der Sclera vorgeschoben wird während dieser Saugnapf verhindert, daß die Nadelspitze die Sclera an der Einstichstelle eindellt. Der Vorschub der Nadelspitze wird hierbei vorzugsweise automatisch mittels eines elektrischen, pneumatischen, hydraulischen oder mechanischen Antriebes bewirkt, so daß die Einrichtung hierbei nicht durch unkontrollierte Bewegungen eventuell das Auge verletzt.

Um einen genau kontrollierbaren Einstich der Nadel zu realisieren, können bei der erfindungsgemäßen Einrichtung sowohl die Ausgangslage der Nadelspitze als auch der Hub der Nadelspitze vor dem Einstich an der Einrichtung genau eingestellt werden.

Um sofort nach dem Einstich das Sclera-Gewebe im Bereich der eingestochenen Nadel mit Gleichstrom verätzen zu können, ist die Nadel außer im Bereich der Nadelspitze und am anderen Ende, wo die elektrisch leitfähige Verbindung der Nadel zur Stromquelle hergestellt wird, elektrisch isoliert.

Um vor, während und/oder nach der Verätzung der Perforation die subretinale Flüssigkeitsansammlung möglichst vollständig zu entfernen, ist die Einrichtung mit einem Absaugkanal ausgestattet, welcher im Bereich des Saugnapfes und der Nadel dadurch gebildet wird, daß der Kanaldurchmesser im Bereich der Nadelführung einen etwas größeren Durchmesser hat als die Nadel, so daß für die Flüssigkeitsabsaugung genügend Platz bleibt.

Ein Ausführungsbeispiel der Einrichtung zur subretinalen Drainage wird im folgenden anhand der schematischen Zeichnungen detaillierter beschrieben. Es zeigen:

Fig. 1 Eine schematische Darstellung eines Teiles eines Augapfels mit Ablatio retinae und angelegtem Saugnapf.

Fig. 2 Eine schematische Darstellung einer erfindungsgemäßen Einrichtung im Längsschnitt.

Fig. 3 Eine schematische Darstellung der erfindungsgemäßen Einrichtung.

Fig. 4 Eine detailiertere Darstellung des elektrischen Anschlusses.

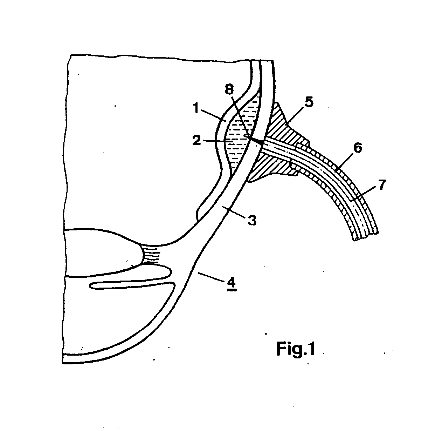

Figur 1 zeigt schematisch eine Netzhautablösung (Ablatio retinae) 1 mit Flüssigkeitsansammlung 2 zwischen der abgelösten Netzhaut 1 und der Sclera 3 des Augapfels 4. Der Saugnapf 5 ist von außen an die Sclera 3 angelegt und infolge der Saugwirkung des Unterdruckes innerhalb des Saugkanales 6 fest an die Sclera 3 angesaugt. In diesem Zustand kann die Nadelelektrode 7 ohne Eindellung der Sclera 3 durch die Sclera 3 hindurchgestochen werden.

Der Saugnapf 5 besteht vorzugsweise aus elektrisch nicht leitendem Material, beispielsweise sterilisierbarem Kunststoff. Die Nadelelektrode 7 ist, außer im vorderen Einstichbereich 8, wo sie die Sclera 3 berührt, elektrisch isoliert. Nach dem Einstich wird zwischen dem Augapfel 4 und der Nadelektrode 7 eine elektrische Gleichspannung, deren positiver Pol an der Nadelelektrode liegt, geschaltet, welche die Sclera im Einstichbereich der Nadelelektrode so verätzt, daß eine längerfristige Perforation entsteht, welche als Drainage für die subretinale Flüssigkeit wirkt.

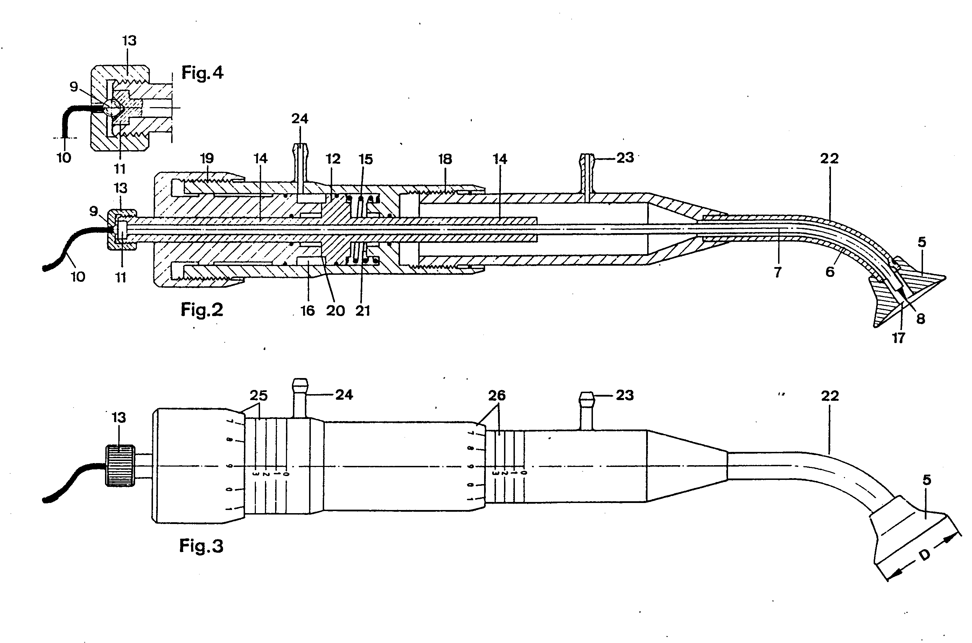

Figur 2 zeigt schematisch ein Schnittbild der gesamten Einrichtung zur subretinalen Drainage. Als Antriebsaggregat wird in diesem Ausführungsbeispiel ein hydraulischer Antrieb verwendet. Die Nadelelektrode 7, welche auf der gesamten Länge elektrisch isoliert ist, außer an der Nadelspitze 8, wie bei Fig. 1 beschrieben, und am anderen Ende, wo die elektrische Verbindung 9 zum Kabel 10 hergestellt wird, ist mit einem Ansatz 11, ähnlich einem Nagelkopf, ausgestattet, mittels welchem sie definiert mit dem Antriebskolben 12 verschraubt ist. Die Überwurfmutter 13 drückt den Kontakt 9 des Kabels 10 auf die nicht isolierte Kuppe des Ansatzes 11 und klemmt gleichzeitig den Ansatz 11 spielfrei gegen die Kolbenstange 14. Die Überwurfmutter 13 besteht aus elektrisch nicht leitfähigem Material. Der Kolben 12 sowie die Kolbenstange 14 besteht vorzugsweise ebenfalls aus elektrisch nicht leitfähigem Material. Um zu gewährleisten, daß die Nadelspitze 8 im Ruhezustand stets in den Saugnapf 5 eingezogen ist, wird der Kolben 12 mittels einer Druckfeder 15 vorgespannt. Der Kolgen 12 wird durch Überdruck in der Kolbenkammer 16 angetrieben.

Die Ruhelage der Nadelspitze 8 relativ zur Austrittsöffnung 17 kann durch das Gewinde 18 justiert werden. Der Hub der Nadelelektrode 7 kann durch das Gewinde 19 justiert werden, wobei die Anschläge 20 und 21 den Hub begrenzen.

Der Außendurchmesser der Nadelelektrode 7 ist etwas kleiner als der Innendurchmesser des vorderen, gebogenen Führungsrohres 22, so daß durch den Zwischenraum eine Saugleitung gebildet wird, welche den Saugnapf 5 mit dem Sauganschlußstutzen 23 verbindet. An den Saugstutzen 23 kann eine Saugeinrichtung über eine flexible Saugleitung angeschlossen werden.

Der Antrieb des Kolbens 12 erfolgt durch Überdruck in der Druckkammer 16. Als Druckmittel können sowohl Gas als auch Flüssigkeiten verwendet werden, die über den Anschlußstutzen 24 zugeführt werden.

Zur Reinigung, Desinfektion oder Sterilisation kann die Einrichtung durch Auseinanderschrauben leicht in die einzelnen Bestandteile zerlegt werden.

In Figur 3 ist die Einrichtung im geschlossenen Zustand dargestellt. Auf den Außendurchmesser bzw. auf den äußeren Umfang sind je eine Skala für den Hub 25 und für die relative Justierung der Lage der Nadelspitze 8 zur Austrittsöffnung 17, dies ist die Skala 26, angebracht. Wird für die Steigung der Gewinde 1 Millimeter pro Umdrehung gewählt, so ist bei einer Skala mit zehn Teilstrichen eine Einstellung des Hubes und der relativen Lage der Nadelspitze 8 auf 0,1 Millimeter möglich.

In weiterer Ausgestaltung der Erfindung kann der Saugnapf 5 austauschbar gestaltet werden, so daß je nach individuellen Gegebenheiten Saugnäpfe mit verschiedenen Außendurchmessern D verwendet werden können.

## Ansprüche

1. Einrichtung zur Drainage subretinaler Flüssigkeitsansammlungen im Auge, wobei mittels einer Nadel ein Loch durch die Sclera gestochen wird, welches anschließend mittels elektrischem Gleichstrom elektrolytisch verätzt wird, **dadurch gekennzeichnet,** daß ein Saugnapf (5), dessen Saugfläche an die Kugelform des Augapfels angepaßt ist, vor dem Einstechen der Nadelspitze (8) der Nadelelektrode (7) an die Außenwand der Sclera (3) angesaugt wird und so eine Eindellung der Sclera (3) während des Einstechens der Nadelspitze (8) verhindert und daß die Einstichtiefe der Nadelspitze (8) durch Voreinstellung der Ausgangslage der Nadelspitze mittels Gewinde (18) und Skala (26) sowie durch Voreinstellung des Hubes der Nadelspitze (8) mittels Gewinde (19) und Skala - (25) justierbar ist und der Antrieb der Nadelelektrode (7) automatisch erfolgt.

2. Einrichtung nach Anspruch 1, **dadurch gekennzeichnet,** daß die Nadelelektrode (7) auf ihrer gesamten Länge elektrisch isoliert ist mit Ausnahme der Nadelspitze (8) und der Stelle, an der die elektrische Verbindung (9) zum Anschlußkabel - (10) vorgesehen ist.

3. Einrichtung nach Anspruch 1 oder 2, **dadurch gekennzeichnet,** daß die Nadelelektrode (7) mechanisch spielfrei mit einem Antriebskolben(12) mittels einer Überwurfmutter - (13) verschraubt ist, wofür die Nadelelektrode (7) mit einem Ansatz (11) ausgestattet ist.

4. Einrichtung nach einem der vorhergehenden Ansprüche, **dadurch gekennzeichnet,** daß der Saugnapf (5) durch Saugnäpfe mit unterschiedlichem Außendurchmesser (D) austauschbar ist.

5. Einrichtung nach einem der vorhergehenden Ansprüche, **dadurch gekennzeichnet,** daß der Hub der Nadelspitze (8) dadurch voreinstellbar ist, daß mechanische Anschläge (20,21) vorhanden sind, welche den Hub des Antriebskolbens (12) begrenzen, wobei der eine Anschlag (20) mittels des zweiten Gewindes (19) justierbar ist.

6. Einrichtung nach einem der vorhergehenden Ansprüche, **dadurch gekennzeichnet,** daß das zweite Gewinde (19) eine Steigung von 1 Millimeter pro Umdrehung hat und daß die zugeordnete Skala (25) vorzugsweise in zehn gleiche Teile unterteilt ist, so daß der Hub der Nadelspitze (8) auf ein Zehntel Millimeter justierbar ist.

7. Einrichtung nach einem der vorhergehenden Ansprüche, **dadurch gekennzeichnet,** daß durch das erste Gewinde (18) die relative Lage der Nadelspitze (8) zur Austrittsöffnung (17) des Saugnapfes (5) justierbar ist.

8. Einrichtung nach Anspruch 7, **dadurch gekennzeichnet,** daß das erste Gewinde (18) vorzugsweise eine Steigung von 1 Millimeter pro Umdrehung hat und daß die zugeordnete Skala (26) vorzugsweise in zehn gleiche Teile unterteilt ist, so daß die relative Lage der Nadelspitze auf ein Zehntel eines Millimeters einjustierbar ist.

9. Einrichtung nach einem der vorhergehenden Ansprüche, **dadurch gekennzeichnet,** daß eine Druckfeder (15) vorhanden ist, welche den Antriebskolben (12) in Ruhestellung gegen den Anschlag (20) drückt, so daß die Nadelspitze (8) in Ruhestellung stets in den Saugnapf (5) hineinbewegt ist.

10. Einrichtung nach einem der vorhergehenden Ansprüche, **dadurch gekennzeichnet,** daß zwischen der Nadelelektrode (7) und dem Innendurchmesser eines vorderen Führungsrohres (22) ein Saugkanal vorhanden ist, welcher mit einem Sauganschlußstutzen (23) in Verbindung steht.

11. Einrichtung nach Anspruch 10, **dadurch gekennzeichnet,** daß das vordere Führungsrohr - (22) gekrümmt ist.

12. Einrichtung nach einem der vorhergehenden Ansprüche, **dadurch gekennzeichnet,** daß der Saugnapf (5) aus elektrisch nicht leitfähigem Material besteht.

5293

Fig.1

Fig.4

Fig.2

Fig.3

0 216 952

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| A | US-A-4 299 227  (LINCOFF) <br> * Spalte 3, Zeilen 54-56 * <br><br> --- | 1 | A 61 F   9/00 |
| A | US-A-2 355 231  (MOORE) <br> *  Seite  2, linke Spalte, Zeilen 62-66 * <br><br> --- | 1 | |
| A | DE-A-  615 278  (VOGEL) <br> * Seite 2, linke  Spalte,  Zeilen 8-12; Figuren * <br><br> --- | 1 | |
| A | SU-A-  175 148  (GOLOWIN et al.) <br> * Insgesamt * <br><br> --- | 1 | |
| A | DE-A-3 215 832  (DOSS) <br> * Anspruch 1; Figuren * <br><br> --- | 1 | |
| A | US-A-1 441 754  (SANDERS) <br> *  Seite  1, Zeile 110 - Seite 2, Zeile 16; Figuren * <br><br> --- | 1 | |
| A | US-A-4 301 802  (POLER) <br> * Spalte 2, Zeilen 38-43,56-62 * <br><br> --- | 1 | |
| A | US-A-3 528 410  (BANKO) <br><br> --- | 1 | |
| A | DE-A-3 425 218   (WALTHER et al.) <br><br> ---          -/- | | |

**KLASSIFIKATION DER ANMELDUNG (Int. Cl. 4)**

A 61 F   9/00

**RECHERCHIERTE SACHGEBIETE (Int. Cl.4)**

A 61 F
A 61 B

Der vorliegende Recherchenbericht wurde für alle Patentansprüche erstellt.

| Recherchenort <br> DEN HAAG | Abschlußdatum der Recherche <br> 20-11-1986 | Prüfer <br> GLAS J. |
|---|---|---|

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-3 321 621 (WIEGERINCK et al.) ----- | | |
| | | | **RECHERCHIERTE SACHGEBIETE (Int. Cl.4)** |

Der vorliegende Recherchenbericht wurde für alle Patentansprüche erstellt.

| Recherchenort DEN HAAG | Abschlußdatum der Recherche 20-11-1986 | Prüfer GLAS J. |
|---|---|---|